# FASCICULE DE BREVET EUROPEEN

(11) **EP 0 458 661 B1**
(45) Date de publication et mention de la délivrance du brevet: **19.03.1997**
(21) Numéro de dépôt: 91400889.1
(22) Date de dépôt: 02.04.1991
(51) Int. Cl.: A61K 31/415, C07D 233/64

(54) **Application thérapeutique d'agonistes du récepteur H3 de l'histamine, nouveaux composés et utilisation pour la préparation de médicaments**
Therapeutische Anwendung von Histamin-H3-Agonisten, neue Wirkstoffe und Verwendung zur Herstellung von Arzneimitteln
Therapeutical application of histamine H3 receptor agonists, new compounds and their use for the preparation of medicaments

(30) Priorité: 13.04.1990 FR 9004861
(43) Date de publication de la demande: 27.11.1991
(73) Titulaire: SOCIETE CIVILE BIOPROJET, F-75003 Paris (FR); INSTITUT NATIONAL DE LA SANTE ET DE LA RECHERCHE MEDICALE (INSERM), 75654 Paris Cédex 13 (FR)
(72) Inventeur: Schwartz, Jean Charles, F-75014 Paris (FR); Garbarg, Monique, F-75017 Paris (FR); Arrang, Jean Michel, F-91190 Gif sur Yvette (FR); Ganellin, Charon Robin, Hertfordshire AL6 OTD (GB); Lecomte, Jeanne Marie, F-75003 Paris (FR)
(74) Mandataire: Bernasconi, Jean

(56) Documents cités:
- EP-A- 0 420 396
- GB-A- 1 296 544
- US-A- 3 759 944
- US-A- 3 954 982
- TRENDS IN PHARMACOLOGICAL SCIENCES, vol. 10, no. 4, April 1989, pages 159-162; J.F. VAN DER WERF et al.: "The histamine H3 receptor: a general presynaptic histaminergic regulatory system?"
- Schayer et al 1970 J. Neurochem. 17;1649-1654

## Description

L'invention concerne l'application thérapeutique de dérivés de l'histamine, un nouveau dérivé de l'histamine et l'utilisation de ces dérivés pour la préparation de médicaments.

La S-[2-(4(5)-imidazolyl)-éthyl] isothiourée (composé I) a déjà été décrite dans le brevet CB-A-1 296 544, dans lequel elle est indiquée comme antagonisant chez l'animal les effets de l'histamine, notamment sur la sécrétion gastrique. Ce composé inhibe la sécrétion d'acide gastrique stimulée par l'histamine dans des estomacs perfusés de rats anesthésiés, à des doses comprises entre 8 et 256 micromoles par kilogramme.

D'après C.R. Ganellin (J. Med. Chem., 1981, 24, 913 et Medicinal Chemistry, chapitre 6, p 93 à 118, Academic Press Inc., London, 1985), ce composé s'oppose à forte dose à l'action de l'histamine sur la sécrétion gastrique du rat : la dose inhibitrice 50 % est de 200 µmoles/kg environ. Selon cet auteur, le composé I n'est pas un antagoniste assez puissant de l'histamine et il est nécessaire de rechercher un composé plus actif.

En outre, Sterk et al. (Agents and Actions, 1986, 18, 137 et Arch. Pharm., 1986, 319, 624) ont décrit ce composé I comme étant un agoniste partiel (50 %) du récepteur H₂ de l'histamine qu'il ne stimule qu'à une concentration très élevée (environ 0,1 mM).

Ces propriétés d'agoniste H₂ partiel et peu puissant suggéraient que le composé n'était pas applicable en thérapeutique, notamment eu égard aux doses élevées qu'il aurait été nécessaire d'administrer, ce qui a conduit à considérer ce composé comme non efficace et à l'écarter de toute étude pharmacologique approfondie.

En 1983, Arrang et al. (Nature, 1983, 302, 832) ont mis en évidence l'existence d'un troisième récepteur de l'histamine appelé H₃.

Or la déposante a découvert, de façon surprenante, que ce composé I constitue un agoniste complet de l'histamine sur le récepteur H₃ qu'il stimule à une concentration proche de 1 nanomolaire, soit environ 100.000 fois plus faible que celles qui sont nécessaires pour une occupation du récepteur H₂. Cette propriété inattendue a été mise en évidence par l'effet inhibiteur que le composé exerce sur la libération et la synthèse d'histamine endogène à partir de coupes de cerveau dépolarisées (selon la technique décrite par Arrang et al., Nature, 1983, 302, 832). A cet égard, ce composé est 62 fois plus puissant que l'histamine et constitue l'agoniste H₃ le plus puissant connu à ce jour.

Qui plus est, ce composé franchit aisément la barrière hémato-encéphalique puisque à la dose de 3 mg/kg par voie orale, il inhibe de manière maximale la synthèse d'histamine dans le cerveau de rat (mesurée selon la technique d'Arrang et al., Nature, 1987, 327, 117). De la même manière, il inhibe à dose très faible la synthèse d'histamine dans divers organes périphériques comme le poumon.

La déposante a également établi que le dérivé N-méthylé correspondant (composé II) de formule 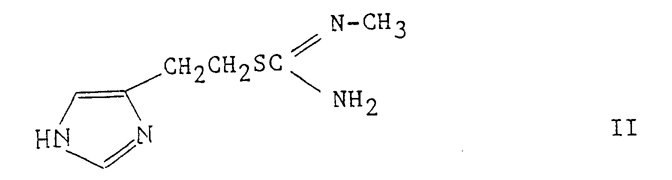 se comporte aussi comme un agoniste H₃ complet et relativement puissant (EC₅₀ = 10 nM) également actif in vivo. Le composé II est environ 6 fois plus puissant que l'histamine.

L'invention a pour objet l'utilisation de ce composé I pour la préparation d'un médicament destiné à être utilisé comme agent tranquillisant, promoteur du sommeil, hypnotique, sédatif, anxiolytique, antiasthmatique, anti-inflammatoire notamment bronchique, cutané, ou oculaire, ou antiulcéreux gastrique, ce composé I étant notamment efficace à une dose comprise entre 0,1 et 10 mg/kg environ, notamment entre 0,3 et 3 mg/kg, de préférence de 1 mg/kg.

L'invention a encore pour objet le composé II dont la formule développée a été donnée ci-dessus.

L'invention a également pour objet un médicament à action agoniste du récepteur H₃ de l'histamine, comprenant le composé II. Celui-ci est notamment efficace à une dose comprise entre 1 et 50 mg/kg environ, notamment entre 3 et 30 mg/kg, de préférence de 10 mg/kg.

Le médicament peut aussi contenir ce dernier composé à une concentration comprise entre 10 nanomoles/l et 1 micromole/l pour des applications topiques cutanées, oculaires ou par aérosol, dans un véhicule approprié.

L'invention a aussi pour objet l'utilisation du composé II pour la réalisation d'un médicament destiné à être utilisé comme agent hypnotique, promoteur du sommeil, tranquillisant, sédatif, anxiolytique, antiasthmatique, anti-inflammatoire, notamment bronchique, cutané ou oculaire, ou antiulcéreux gastrique. Ce composé II est notamment efficace à une dose comprise entre 1 et 50 environ, notamment entre 3 et 30 mg/kg, de préférence de 10 mg/kg.

Les composés I et II pourront être administrés à l'homme en association avec un excipient ou véhicule pharmaceutiquement acceptable, à titre de tranquillisant, de promoteur du sommeil, d'agent hypnotique, sédatif, anxiolytique, antiasthmatique, anti-inflammatoire, notamment bronchique, oculaire ou cutané, ou antiulcéreux gastrique.

Les composés I et II peuvent être notamment préparés par le procédé décrit dans le brevet GB-A-1.296.544 ou des procédés similaires.

Pour la préparation des médicaments selon l'invention, on mélange le composé, dosé, à des excipients et véhicules usuels pour les administrations orales, parentérales ou topiques envisagées.

## Revendications

1. Utilisation de la S-[2-(4(5)-imidazolyl)-éthyl] isothiourée pour la préparation d'un médicament à action agoniste du récepteur H₃ de l'histamine destiné à être utilisé comme agent hypnotique, promoteur du sommeil, tranquillisant, sédatif, anxiolytique, anti-inflammatoire, notamment bronchique,cutané ou oculaire ou antiulcéreux gastrique.

2. Utilisation de la S-[2-(4(5)-imidazolyl)-éthyl] isothiourée pour la préparation d'un médicament à action agoniste du récepteur H₃ de l'histamine efficace à raison d'une dose du composé comprise entre 0,1 et 10 mg/kg environ, notamment entre 0,3 et 3 mg/kg, de préférence d'environ 1 mg/kg comme agent hypnotique, promoteur du sommeil, sédatif, tranquillisant, anxiolytique, anti-inflammatoire, notamment bronchique, cutané ou oculaire, ou antiulcéreux gastrique.

3. Composé de formule

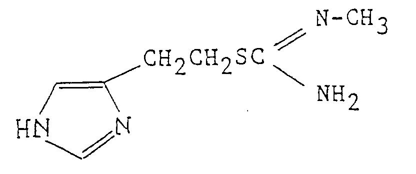

4. Médicament à action agoniste du récepteur H₃ de l'histamine, comprenant le composé de la revendication 5.

5. Médicament à action agoniste du récepteur H₃ de l'histamine, comprenant le composé de la revendication 3 efficace à raison d'une dose comprise entre 1 et 50 mg/kg environ, notamment entre 3 et 30 mg/kg, de préférence de 10 mg/kg.

6. Utilisation du composé de la revendication 3 pour la préparation d'un médicament destiné à être utilisé comme agent hypnotique, promoteur du sommeil, tranquillisant, sédatif, anxiolytique, antiasthmatique, anti-inflammatoire, notamment bronchique, cutané ou oculaire, ou antiulcéreux gastrique.

7. Utilisation du composé de la revendication 3 pour la préparation d'un médicament efficace à raison d'une dose du composé comprise entre 1 et 50 mg/kg environ, notamment entre 3 et 30 mg/kg, de préférence de 10 mg/kg comme agent hypnotique, promoteur du sommeil, tranquillisant, sédatif, anxiolytique, antiasthmatique, anti-inflammatoire, notamment bronchique, cutané ou oculaire, ou antiulcéreux gastrique.

## Claims

1. Use of S-[2-(4(5)-imidazolyl)-ethyl] isothiourea for the preparation of a medicament with histamine H₃ receptor agonist action intended to be used as a hypnotic agent, an agent for promoting sleep, a tranquillising agent, sedative agent, anxiolytic agent, anti-inflammatory agent, in particular bronchial, cutaneous or ocular agent or an agent for treating gastric ulcers.

2. Use of S-[2-(4(5)-imidazolyl)-ethyl] isothiourea for the preparation of a medicament with histamine H₃ receptor agonist action effective at a rate of a dose of the compound comprised between 0.1 and 10 mg/kg approximately, in particular between 0.3 and 3 mg/kg, preferably of approximately 1 mg/kg as a hypnotic agent, an agent promoting sleep, as a sedative agent, tranquillising agent, anxiolytic agent, anti-inflammatory agent, in particular bronchial, cutaneous or ocular agent, or as an agent for the treatment of gastric ulcers.

3. Compound of formula

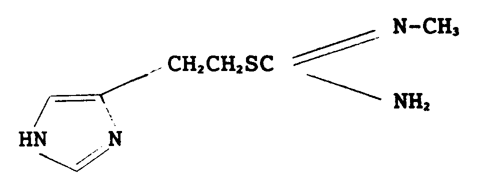

4. Medicament with histamine H₃ receptor agonist action, comprising the compound of Claim 5.

5. Medicament with histamine H₃ receptor agonist action, comprising the compound of Claim 3 effective at a rate of a dose comprised between 1 and 50 mg/kg approximately, in particular between 3 and 30 mg/kg, preferably of 10 mg/kg.

6. Use of the compound of Claim 3 for the preparation of a medicament intended to be used as a hypnotic agent, an agent promoting sleep, tranquillising agent, sedative, anxiolytic agent, anti-asthmatic agent, anti-inflammatory agent, in particular bronchial, cutaneous or ocular agent, or as an agent for the treatment of gastric ulcers.

7. Use of the compound of Claim 3 for the preparation of a medicament effective at a rate of a dose of the compound comprised between 1 and 50 mg/kg approximately, in particular between 3 and 30 mg/kg, preferably of 10 mg/kg as a hypnotic agent, as an agent for promoting sleep, as a tranquillising agent, sedative, anxiolytic agent, anti-asthmatic agent, anti-inflammatory agent, in particular bronchial, cutaneous or ocular agent, or as an agent for the treatment of gastric ulcers.

## Patentansprüche

1. Verwendung von S-[2-(4(5)-Imidazolyl)ethyl]-isothioharnstoff für die Herstellung eines Medikamentes mit agonistischer Wirkung auf den H₃-Rezeptor von Histamin, das dazu bestimmt ist, als hypnotisches Mittel, schlafförderndes Mittel, Tranquilizer, Sedativum, anxiolytisches Mittel, insbesondere bronchiales, kutanes oder okulares entzündungshemmendes Mittel oder Magengeschwür-hemmendes Mittel verwendet zu werden.

2. Verwendung von S-[2-(4(5)-Imidazolyl)ethyl]-isothioharnstoff für die Herstellung eines Medikamentes mit agonistischer Wirkung auf den H₃-Rezeptor von Histamin, das in einer Dosismenge der Verbindung zwischen ungefähr 0,1 und 10 mg/kg, insbesondere zwischen 0,3 und 3 mg/kg, vorzugsweise von ungefähr 1 mg/kg, als hypnotisches Mittel, schlafförderndes Mittel, Sedativum, Tranquilizer, anxiolytisches Mittel, insbesondere bronchiales, kutanes oder okulares entzündungshemmendes Mittel oder Magengeschwür-hemmendes Mittel wirksam ist.

3. Verbindung der Formel

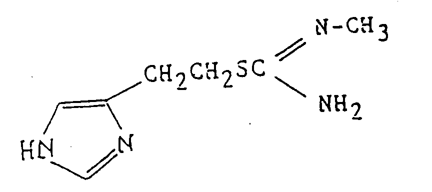

4. Medikament mit agonistischer Wirkung auf den H₃-Rezeptor von Histamin, umfassend die Verbindung von Anspruch 3.

5. Medikament mit agonistischer Wirkung auf den H₃-Rezeptor von Histamin, umfassend die Verbindung von Anspruch 3, die in einer Dosismenge zwischen ungefähr 1 und 50 mg/kg, insbesondere zwischen 3 und 30 mg/kg, vorzugsweise von 10 mg/kg, wirksam ist.

6. Verwendung der Verbindung des Anspruchs 3 bei der Herstellung eines Medikamentes, das dazu bestimmt ist, als hypnotisches Mittel, schlafförderndes Mittel, Tranquilizer, Sedativum, anxiolytisches Mittel, antiasthmatisches Mittel, insbesondere bronchiales, kutanes oder okulares entzündungshemmendes Mittel oder als Magengeschwür-hemmendes Mittel verwendet zu werden.

7. Verwendung der Verbindung von Anspruch 3 für die Herstellung eines Medikamentes, das in einer Dosismenge der Verbindung zwischen ungefähr 1 und 50 mg/kg, insbesondere zwischen 3 und 30 mg/kg, vorzugsweise von 10 mg/kg, als hypnotisches Mittel, schlafförderndes Mittel, Tranquilizer, Sedativum, anxiolytisches Mittel, antiasthmatisches Mittel, insbesondere bronchiales, kutanes oder okulares entzündungshemmendes Mittel oder als Magengeschwür-hemmendes Mittel wirksam ist.
